(19) Europäisches Patentamt
European Patent Office
Office européen des brevets

(11) EP 0 713 856 B1

(12) EUROPÄISCHE PATENTSCHRIFT

(45) Veröffentlichungstag und Bekanntmachung des Hinweises auf die Patenterteilung:
**08.09.1999 Patentblatt 1999/36**

(51) Int. Cl.[6]: **C07C 63/70**, C07C 51/265, C07C 65/34

(21) Anmeldenummer: **95117558.7**

(22) Anmeldetag: **08.11.1995**

# (54) Verfahren zur Herstellung bromierter oder chlorierter aromatischer Carbonsäuren

Process for preparing brominated or chlorinated aromatic carboxylic acids

Procédé de préparation d'acides aromatiques carboxyliques bromés ou chlorés

(84) Benannte Vertragsstaaten:
**BE CH DE FR GB LI NL SE**

(30) Priorität: **24.11.1994 DE 4441881**

(43) Veröffentlichungstag der Anmeldung:
**29.05.1996 Patentblatt 1996/22**

(73) Patentinhaber: **Clariant GmbH**
**65929 Frankfurt am Main (DE)**

(72) Erfinder:
- **Röhrscheid, Freimund, Dr.**
  **D-65779 Kelkheim (DE)**
- **Grötsch, Georg, Dr.**
  **D-65812 Bad Soden (DE)**

(56) Entgegenhaltungen:
**EP-A- 0 121 684**
**DE-A- 1 468 858**
**DE-C- 3 440 650**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 713 856 B1

## Beschreibung

Die Erfindung betrifft ein Verfahren zur Herstellung bromierter oder chlorierter aromatischer Carbonsäuren durch Oxidation der entsprechenden Alkylaromaten.

Es ist bekannt, daß sich Alkylaromaten mit Luftsauerstoff in Essigsäure bei Temperaturen oberhalb 120°C mit hoher Ausbeute zu den entsprechenden Benzoesäuren oder Oligocarbonsäuren oxidieren lassen, wenn man die Reaktion durch Bromide der Nebengruppenelemente Kobalt und Mangan (US 2.833.816) oder deren Kombinationen mit Nickel (US 3.334.135), Eisen (DE 2.749.638) oder Zirkon (DE 2.420.960) katalysiert.

Die Oxidation der Lösungen von Alkylbenzolen in Essigsäure mit Luftsauerstoff zu den entsprechenden Benzoesäuren oder aromatischen Oligocarbonsäuren läßt sich ökonomisch und ökologisch besonders günstig durchführen, wenn die Mutterlauge des vorhergehenden Ansatzes wieder als Reaktionsmedium verwendet werden kann; denn diese Mutterlauge enthält den Katalysator, einen Teil des Produkts und Vorstufen des Zielprodukts (volle Ausbeute erst bei Rückführung der Mutterlauge).

Die Oxidation halogenierter Alkylaromaten liefert unter den o.g. Bedingungen die entsprechenden Carbonsäuren mit hoher Ausbeute (EP 002 749). Bei der Oxidation bromierter oder chlorierter Alkylaromaten wie z.B. Chlortoluol, Dichlortoluol, Bromchlortoluol, 2-Chlor-1,4-dimethylbenzol oder 2,4-Dimethyl-2'-chlorbenzophenon tritt aber das Problem auf, daß sich ein geringer Prozentsatz (ca. 1 - 3 %) des Halogens vom Aromaten abspaltet und in der Lösung als Halogenid oder Halogenwasserstoff vorliegt.

Dies führt zu Korrosionsproblemen. Zur Verhinderung der Korrosion des Reaktors wurde als Maßnahme in der EP 121 684 die Zugabe von Alkaliacetaten in einer wäßrig verdünnten Essigsäure beschrieben. Diese Bedingungen vermindern zwar die Korrosion des Reaktors, aber die entstandenen Alkalihalogenide NaCl, NaBr, KCl, KBr besitzen in wäßriger Essigsäure, auch bei geringem, z.B. 1 - 5 %igen Wassergehalt eine beträchtliche Löslichkeit. Dadurch kommt es zu einer starken Anreicherung von Alkalihalogenid in der mehrfach rezyklisierten Mutterlauge. Hohe Alkalihalogenidkonzentrationen wirken aber wieder korrodierend auf Edelstähle und führen außerdem zur vermehrten Bildung von störendem Benzylhalogenid in der Produktlösung` Dieses ist für eine technische Anwendung der Oxidationsreaktion von großem Nachteil, weil die Benzylhalogenide in das Produkt eingelagert werden und ihre Anreicherung nur eine reduzierte Zahl von Mutterlaugenrückführungen zuläßt. Außerdem besitzen Benzylhalogenide einen erheblichen Geruch und wirken tränenreizend.

In der EP 121 684 wird kein Weg gewiesen, wie das sich anreichernde Alkalihalogenid aus dem Kreislauf ausgeschleust werden kann. In der Patentschrift wird vielmehr explizit ein Wassergehalt > 1 Gew.-% beschrieben, was die Anreicherung begünstigt, um eine möglichst hohe Ausbeute zu erzielen.

Es bestand somit ein großer Bedarf nach einem Verfahren das diese Nachteile vermeidet und es erlaubt, die chlorierten und bromierten aromatischen Carbonsäuren in hoher Ausbeute ohne Verunreinigung durch die entsprechenden Arylmethylhalogenide herzustellen.

Diese Aufgabe wird gelöst durch ein Verfahren zur Herstellung von brom-oder chlorsubstituierten aromatischen Carbonsäuren durch Oxidation der entsprechend halogenierten Alkylaromaten mit Sauerstoff oder einem Sauerstoff enthaltenden Gas im Lösemittel Essigsäure und in Gegenwart eines Übergangsmetall-Bromid-Katalysators dadurch gekennzeichnet, daß

a) die Reaktionsmischung ein Natrium- und/oder Kaliumsalz einer schwachen Säure enthält,
b) nach Beendigung der Reaktion die Reaktionslösung abgekühlt und das kristallisierte Produkt aus der Reaktionslösung abfiltriert wird,
c) die vereinigten Mutter- und Waschlaugen destillativ auf einen Wassergehalt ≤ 1,0 % entwässert werden,
d) das ausgefallene Alkalihalogenid aus der Mutterlauge abfiltriert wird und
e) die Mutterlauge als Reaktionsmedium wiederverwendet wird.

Bei dieser Verfahrensweise können die in sehr guter Ausbeute von üblicherweise 90 - 99 % (Rückführung der Mutterlauge) entstandene Carbonsäure und das Alkalihalogenid getrennt und ohne großen Aufwand isoliert werden. Die Verfahrensweise nutzt die Beobachtung, daß die Löslichkeit der Salze NaCl, KCl, NaBr, KBr in der Mutterlauge bei einem Wassergehalt unterhalb 3 Gew.-%, insbesondere unterhalb 1,0 Gew.-% sehr niedrig wird. Außerdem wurde überraschend gefunden, daß in einer Mutterlauge mit weniger als 1,0 % Wasser das KBr deutlich weniger löslich ist als NaBr; und NaCl deutlich weniger löslich ist als NaBr. Unter Anwendung der nachfolgend geschilderten Verfahrensweise ist es möglich, störende Halogenidionen zu entfernen und in der Mutterlauge eine konstant niedrige Konzentration dieses Alkalihalogenids zu erreichen und selektiv Chloridionen neben Bromidionen zu fällen. Der als Katalysator benötigte Anteil an Bromidionen bleibt demgemäß weitgehend erhalten.

Die folgende Verfahrensweise hat sich in vielen Fällen bewährt:

a) Die Oxidation des halogensubstituierten Alkylaromaten erfolgt in Gegenwart des Natrium- oder Kaliumsalzes

einer schwachen Säure, wie z.B. Natrium- oder Kaliumacetat. Die molare Menge dieses Alkalisalzes wird vorteilhaft größer gewählt als die molare Halogenidmenge, die sich zusammensetzt aus dem Cokatalysator Bromid und der Halogenidmenge, die während der Oxidation aus dem eingesetzten halogensubstituierten Alkylaromaten abgespalten wird.
Die Natrium- oder Kaliumsalze der schwachen Säuren können als Salze von aliphatischen oder aromatischen Carbonsäuren zugegeben werden.
Als aliphatische Säuren können z.B. Essigsäure, Propionsäure, Buttersäure als aromatische Carbonsäuren die Benzoesäure oder substituierte Benzoesäuren verwendet werden. Es ist auch möglich Natrium- oder Kaliumcarbonat oder -hydroxid zuzugeben, woraus sich mit der vorhandenen Essigsäure Natrium- oder Kaliumacetat bildet.
b) Nach beendeter Reaktion wird die Reaktionslösung auf 10 bis 60°C, insbesondere 20 bis 30°C, abgekühlt. Das dadurch auskristallisierte Produkt wird abfiltriert und mit Essigsäure gewaschen.
c) Von den vereinigten Filtraten wird in einer Destillationskolonne das bei der Reaktion entstandene Wasser abdestilliert, bis ein Wassergehalt von weniger als 3 %, insbesondere weniger als 1 % in der Mutterlauge eingestellt ist.
d) Die ausgefallenen Alkalihalogenide werden von der Mutterlauge abfiltriert.
e) Die ggf. aufgetretenen Katalysatorverluste der Mutterlauge werden ergänzt. Die Mutterlauge wird in den Oxidationsreaktor zurückgeführt und als Reaktionsmedium für die Oxidation des halogenierten Alkylaromaten wieder verwendet. Die für einen Oxidationsansatz erforderliche Menge des Alkalisalzes der schwachen Säure kann der Reaktionslösung vor der Oxidation, Schritt(e), oder auch schon der Mutterlauge vor der Filtration (d) oder zu Beginn der Destillation (c) zugeführt werden.
Zur Entfernung von Chloridionen, wie sie z.B. bei der Oxidation von Chloraromaten entstehen, fügt man vorzugsweise das Natriumsalz einer schwachen Säure, z.B. Natriumacetat zu, weil dadurch NaCl ausfällt, der Katalysatorbestandteil Bromid aber größtenteils in Lösung bleibt.
Zur Fällung von Bromidionen, wie sie bei der Oxidation von Bromaromaten entstehen, verwendet man vorzugsweise das Kaliumsalz einer schwachen Säure, z.B. Kaliumacetat, weil Kaliumbromid im Gegensatz zu Natriumbromid in essigsaurer Mutterlauge mit niedrigem Wassergehalt sehr wenig löslich ist.

Als Übergangsmetall-Bromid Katalysator werden mit gutem Erfolg Verbindungen eingesetzt, die eines oder mehrere der Metalle Cobalt, Mangan, Zirkon oder Nickel enthalten. In vielen Fällen bewährt haben sich Katalysatoren die Cobalt enthalten.

Das Verfahren ist generell zur Herstellung von chlorierten oder bromierten aromatischen Carbonsäuren geeignet. Als Aromaten sind alle aromatischen Verbindungen zu verstehen, die unter den Reaktionsbedingungen stabil sind, z.B. Phenyl oder Naphthyl.
Neben Chlor und/oder Brom können die Aromaten auch noch einen oder mehrere, gleiche oder verschiedene Substituenten tragen, wie z.B Fluor-, Aryl-, Nitro-, Alkoxy-, Aryloxy-, tert. Alkyl-, $SO_2$-Alkyl-, $SO_2$-Phenyl, Keto-, Phosphonsäure-, Phosphonoxid, $SO_2$-$NH_2$, Triorganylsilyl, perfluorierte Alkylgruppen.

Wichtig ist das Verfahren zur Herstellung von Verbindungen der Formel (I)

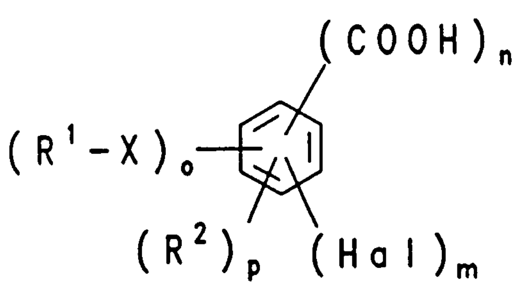

(I)

worin bedeuten:

- X: Einfachbindung, Sauerstoff, C = O, $SO_2$, $C(CF_3)_2$, $C(CH_3)_2$, PO-($C_1$-$C_6$)-Alkyl, PO-Phenyl, Si-($C_1$-$C_6$-Alkyl)$_2$, Si-($C_1$-$C_6$-Alkyl)-Phenyl, Si-(Phenyl)$_2$, -$N_2$-
- $R^1$: substituiertes oder unsubstituiertes Phenyl, Naphthyl,
- Hal: Chlor, Brom
- $R^2$: Wasserstoff, O-($C_1$-$C_6$)-Alkyl, tert.-Alkyl, $SO_2$-($C_1$-$C_6$)-Alkyl, $SO_2$-$NH_2$, $NO_2$, F, PO-[($C_1$-$C_6$)-Alkyl]$_2$, PO(OH)-($C_1$-$C_6$)-Alkyl, PO-(OH)$_2$
- n, m: eine ganze Zahl zwischen 1 und 4,

o, p Null oder 1, wobei $n + m + o + p \leq 6$ .

Von besonderem Interesse ist das Verfahren zur Herstellung von Chlorbenzoesäuren, Dichlorbenzoesäuren, Fluorbenzoesäuren, Brombenzoesäuren, chlorierten und bromierten Benzophenoncarbonsäuren, chlorierten und bromierten Biphenylcarbonsäuren.

Die folgenden Beispiele sollen das Verfahren erläutern, ohne es darauf zu beschränken.

Beispiele

Die Oxidationen werden in einem beheizbaren 1 l-Autoklav durchgeführt, der mit Rührer, Gaseinleitrohr, Rückflußkühler und einer Sauerstoffmessung ausgerüstet ist.

Beispiel 1: Oxidation von 4-Chlortoluol

150,0 g 4-Chlortoluol, 500 g Eisessig, 10,0 g Kobaltacetattetrahydrat, 1,54 g Natriumbromid und 2,46 g Natriumacetat werden in den Autoklaven gefüllt und unter 15 bar Stickstoff auf 155°C geheizt. Dann werden Luft eingeleitet, Temperatur und Druck auf 160°C/166 bar geregelt und der Luftstrom so eingestellt, daß der Sauerstoffrestgehalt im Abgas 1 - 3 Vol-% beträgt. Die stark exotherme Reaktion ist nach 30 Minuten beendet. Nach weiteren 20 Minuten wird Stickstoff eingeleitet, der Reaktor abgekühlt und bei 110°C die Produktlösung entnommen.

Die Produktlösung wird unter Rühren auf 20°C abgekühlt. Das Kristallisat wird auf einem Saugfilter abgetrennt, mit 270 g Eisessig gewaschen und getrocknet. Ausbeute: 167,3 g 4-Chlorbenzoesäure (90,2 % d.Th.), Fp. 239-240°C.

Die vereinigten Filtrate, genannt "Mutterlauge" (744 g) enthalten 27,6 g (3,7 Gew.-%) $H_2O$, 0,48 g Chlorid-, 0,60 g Bromid- und 0,97 g Natriumionen.

1. Rezyklisierung

Die Mutterlauge wird an einer Destillationskolonne auf 510 g konzentriert, wobei der Wassergehalt auf 0,3 Gew.-% zurückgeht. In der Lösung ist kein Niederschlag erkennbar.
Oxidation: Zur konzentrierten Mutterlauge werden 0,28 g Natriumbromid, 1,15 g Natriumacetat und 150 g 4-Chlortoluol hinzugefügt. Die Mischung wird in der oben beschriebenen Weise umgesetzt und aufgearbeitet.
Ausbeute: 179,3 g (96,6 % d.Th.) 4-Chlorbenzoesäure, Fp. 239-241°C.
Die Mutterlauge (737 g) enthält 26,9 g (3,6 Gew.-%) Wasser, 0,95 g Chlorid-, 0,45 g Bromid und 1,92 g Natriumionen.

2. Rezyklisierung

Die Mutterlauge wird nach Zugabe von 1,15 g (0,014 Mol) Natriumacetat auf 510 g Lösung mit 0,4 Gew.-% Wasser konzentriert. Nach dem Abkühlen auf 20°C wurden 0,46 g Natriumchlorid abfiltriert.
Oxidation: Die Mischung aus der konzentrierten Mutterlauge, 150 g 4-Chlortoluol und 0,30 g Natriumbromid wird wie oben beschrieben oxidiert.
Ausbeute: 197,5 g (96,7 % d.Th.) Fp. 238-240°C.
Die Mutterlauge (734 g) enthält 27,2 g (3,7 Gew.-%) Wasser, 1,17 g Chlorid-, 0,68 g Bromid- und 2,06 g Natriumionen.

Nachfolgende Rezyklisierungen

Nach Zugabe von 1,15 g Natriumacetat wird die Mutterlauge auf 510 g mit 0,25 % Wasser konzentriert. Die Filtration bei 20°C liefert 0,78 g Natriumchlorid.
Die durchschnittliche Ausbeute der nachfolgenden Oxidationen ist 182,5 g (97,9 % d.Th.). Der Festpunkt ist konstant 238-240°C. Die Menge des isolierten Natriumchlorids hält sich bei den nachfolgenden Mutterlaugenkonzentrationen auf dem Niveau der 3. Rezyklisierung:
ca. 0,80 g NaCl pro Ansatz.
Die in der Mutterlauge gelöste Chloridmenge stellt sich auf einen Bereich von $1{,}2 \pm 0{,}2$ g ein.
Das ausgefallene Natriumchlorid enthält nur geringe Mengen Natriumbromid (7 Mol-%).

Beispiel 2 Oxidation von 2-Bromtoluol

256,6 g 2-Bromtoluol, 500 g Eisessig, 7,5 g Kobaltacetattetrahydrat, 2,5 g Manganacetattetrahydrat, 1,03 g Natriumbromid, 2,46 g Natriumacetat und 2,94 g Kaliumacetat werden auf gleiche Weise wie in Beispiel 1 bei 155°C

und 16 bar im Laufe von 40 Minuten oxidiert. Das Kristallisat aus der auf 20°C abgekühlten Produktlösung wird abgesaugt, dreimal mit je 70 g Eisessig gewaschen und getrocknet.
Ausbeute: 178,0 g 2-Brombenzoesäure (59,0 % d.Th.), Fp. 148-149°C.

Die vereinigten Filtrate, genannt "Mutterlauge" (761 g) enthalten 34,2 g (4,5 Gew.-%) Wasser, 3,32 g Bromid-, 0,87 g Natrium- und 1,17 g Kaliumionen.

1. Rezyklisierung

Die Mutterlauge wird auf 530 g mit 0,2 Gew.-% $H_2O$ konzentriert. Aus der Lösung (60°C) werden 2,02 g Kaliumbromid abfiltriert. 256,6 g 2-Bromtoluol werden mit der konzentrierten Mutterlauge und 2,94 g (0,03 Mol) Kaliumacetat vermischt und auf die oben beschriebene Weise oxidiert und aufgearbeitet. Ausbeute: 279,6 g 2-Brombenzoesäure (92,7 % d.Th.).
Die Mutterlauge (810 g) enthält 32,4 g (4,0 Gew.-%) Wasser, 3,64 g Bromid-, 0,83 g Natrium- und 1,68 g Kaliumionen.

2. Rezyklisierung

Die Mutterlauge wird auf 521 g mit 0,3 Gew.-% Wasser konzentriert. Durch Filtration bei 60°C werden 3,87 g KBr isoliert. 256,6 g 2-Bromtoluol werden mit der konzentrierten Mutterlauge und 3,81 g Kaliumacetat vermischt und auf die oben beschriebene Weise oxidiert und aufgearbeitet.
Ausbeute: 281,2 g 2-Brombenzoesäure (93,2 % d.Th.).
Die Mutterlauge (803 g) enthält 32,9 g (4,1 Gew.-%) Wasser, 3,46 g Bromid-, 0,77 g Natrium- und 1,56 g Kaliumionen.

Nachfolgende Rezyklisierungen

Die Mutterlauge wird auf 529 g mit 0,55 % Wasser konzentriert. Aus der Lösung werden 3,37 g Kaliumbromid abfiltiert. Die konzentrierte Mutterlauge wird auf die beschriebene Weise wieder als Reaktionsmedium für die Oxidation eingesetzt.
In den nachfolgenden Oxidationen liegt die durchschnittliche Ausbeute für 2-Brombenzoesäure bei 93,8 % d.Th.
Die Menge des isolierten Kaliumbromids hält sich in der Größenordnung von 3,7 g pro Ansatz.
Dem entsprechend werden 3,1 g (0,0316 Mol) Kaliumacetat pro Ansatz zugefügt.
Die Bromidionenmenge in den Mutterlaugen wird auf diese Weise unterhalb 3,6 g gehalten.
Das ausgefallene Kaliumbromid enthält nur geringe Mengen Natriumbromid.

Beispiel 3 Oxidation von 2,3-Dichlortoluol

193,2 g 2,3-Dichlortoluol, 400 g Eisessig, 2,96 g Kobaltacetat Tetrahydrat, 0,96 g Manganacetat Tetrahydrat, 1,65 g Natriumbromid, 1,97 g Natriumacetat werden wie im Beispiel 1 im Laufe von 2 Stunden bei 155-160°C und 15-16 bar mit Luft oxidiert. die kristallisierte Dichlorbenzoesäure wird abfiltriert und getrocknet. Farblose Kristallnadeln, Fp. 166,0-168,5°C.

Die Mutterlauge wird wie im Beispiel 1 als Reaktionsmedium für die nachfolgenden Ansätze á 154,6 g 2,3-Dichlortoluol verwendet.
Bei den Rezyklisierungen werden pro Ansatz 1,15 g Natriumacetat und 0,62 g Natriumbromid zugefügt und 0,82 g Natriumchlorid ausgeschleust. Es entstehen 0,014 Mol Chlorid pro Mol 2,3-Dichlortoluol.

Die Selektivität für 2,3-Dichlorbenzoesäure ist 96,9 %.

Beispiel 4 Oxidation von 4-Chlor-4'-methylbenzophenon

279,0 g 4-Chlor-4'methylbenzophenon, 1000 g Eisessig, 15,0 g Kobaltacetat Tetrahydrat, 1,23 g Natriumbromid und 0,82 g Natriumacetat werden wie im Beispiel 1 im Laufe einer Stunde bei 175°C und 16 bar mit Luft oxidiert. Die Chloridabspaltung beträgt 0,4 Mol-%.
Pro Rezyklisierung werden 0,40 g Natriumacetat und 0,33 g HBr in Form von 1,0 g einer Lösung von 33 Gew.-% HBr in Eisessig zugefügt.
Die Selektivität von 4'-Chlor-benzophenon-4-carbonsäure ist 97,4 %, Fp. 253-255°C.

## Patentansprüche

1. Verfahren zur Herstellung von brom- oder chlorsubstituierten aromatischen Carbonsäuren durch Oxidation der entsprechend halogenierten Alkylaromaten mit Sauerstoff oder einem Sauerstoff enthaltenden Gas im Lösungsmittel

Essigsäure und in Gegenwart eines Übergangsmetall-Bromid-Katalysators, dadurch gekennzeichnet, daß

a) die Reaktionsmischung ein Natrium- und/oder Kaliumsalz einer schwachen Säure enthält,
b) nach Beendigung der Reaktion die Reaktionslösung abgekühlt und das kristallisierte Produkt aus der Reaktionslösung abfiltriert wird,
c) die vereinigten Mutter- und Waschlaugen destillativ auf einen Wassergehalt ≤ 1,0 % entwässert werden,
d) das ausgefallene Alkalihalogenid aus der Mutterlauge abfiltriert wird und
e) die Mutterlauge als Reaktionsmedium wiederverwendet wird.

**2.** Verfahren nach Anspruch 1, dadurch gekennzeichnet, daß das Natrium oder Kaliumsalz der schwachen Säure vor der Reaktion, oder bei Rezyklisierung der Mutterlauge vor der Filtration (d) oder der Entwässerung (c) zugefügt wird.

**3.** Verfahren nach Anspruch 1 oder 2, dadurch gekennzeichnet, daß die Natrium oder Kaliumsalze der schwachen Säure als Salze von aliphatischen oder aromatischen Carbonsäuren, insbesondere Essigsäure, Propionsäure, Buttersäure oder Benzoesäure zugegeben werden.

**4.** Verfahren nach Anspruch 1 oder 2, dadurch gekennzeichnet, daß die Natrium- und Kaliumsalze der schwachen Säure im Reaktionsmedium Natrium- oder Kaliumacetat sind, die durch Zugabe der Natrium- bzw. Kaliumcarbonate oder -hydroxide zum Lösungsmittel Essigsäure gebildet werden.

**5.** Verfahren nach Anspruch 1 oder 2, dadurch gekennzeichnet, daß zur Oxidation von chlorierten Alkylaromaten vorzugsweise Natriumacetat zugefügt wird.

**6.** Verfahren nach Anspruch 1 oder 2, dadurch gekennzeichnet, daß zur Oxidation von bromierten Alkylaromaten vorzugsweise Kaliumacetat zugefügt wird.

**7.** Verfahren nach mindestens einem der Ansprüche 1 bis 6, dadurch gekennzeichnet, daß als Übergangsmetall-Bromid-Katalysator eine Verbindung eingesetzt wird, die eines oder mehrere der Metalle Cobalt, Mangan, Zirkon oder Nickel, insbesondere Cobalt, enthält.

**8.** Verfahren nach mindestens einem der Ansprüche 1 bis 7, dadurch gekennzeichnet, daß nach Beendigung der Reaktion die Reaktionslösung auf 10 bis 60°C, insbesondere 20 bis 30°C, abgekühlt wird.

**9.** Verfahren nach mindestens einem der Ansprüche 1 bis 8, dadurch gekennzeichnet, daß als aromatische Verbindungen substituierte oder unsubstituierte Phenyle oder Naphthyle eingesetzt werden.

**10.** Verfahren nach mindestens einem der Ansprüche 1 bis 9, dadurch gekennzeichnet, daß als aromatische Carbonsäuren Verbindungen der Formel (I)

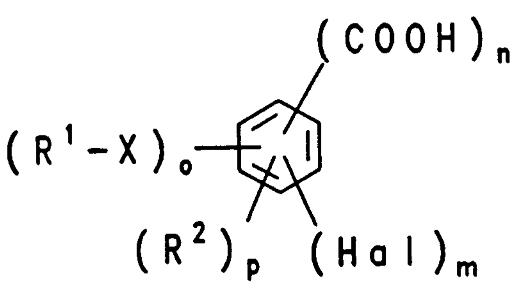

(I)

worin bedeuten:

X Einfachbindung, Sauerstoff, C = O, $SO_2$, $C(CF_3)_2$, $C(CH_3)_2$, PO-($C_1$-$C_6$)-Alkyl, PO-Phenyl, Si-($C_1$-$C_6$-Alkyl)$_2$, Si-($C_1$-$C_6$-Alkyl)-Phenyl, Si-(Phenyl)$_2$, -$N_2$-
$R^1$ substituiertes oder unsubstituiertes Phenyl, Naphthyl,
Hal Chlor, Brom
$R^2$ Wasserstoff, O-($C_1$-$C_6$)-Alkyl, tert.-Alkyl, $SO_2$-($C_1$-$C_6$)-Alkyl, $SO_2$-$NH_2$, $NO_2$, F, PO-[($C_1$-$C_6$)-Alkyl]$_2$,

PO(OH)-($C_1$-$C_6$)-Alkyl, PO-$(OH)_2$ n, m eine ganze Zahl zwischen 1 und 4, wobei $n + m \leq 5$ ,
o, p Null oder 1, wobei $n + m + o + p \leq 6$
hergestellt werden.

**11.** Verfahren nach mindestens einem der Ansprüche 1 bis 8, dadurch gekennzeichnet, daß Chlorbenzoesäuren, Dichlorbenzoesäuren, Fluorbenzoesäuren, Brombenzoesäuren, chlorierte und bromierte Benzophenoncarbonsäuren, chlorierte und bromierte Biphenylcarbonsäuren hergestellt werden.

## Claims

**1.** A process for the preparation of a bromine- or chlorine-substituted aromatic carboxylic acid by oxidation of the correspondingly halogenated alkylaromatic with oxygen or an oxygen-containing gas in the solvent acetic acid and in the presence of a transition metal bromide catalyst, which comprises a procedure in which

a) the reaction mixture comprises a sodium and/or potassium salt of a weak acid,
b) when the reaction has ended, the reaction solution is cooled and the product which has crystallized is filtered off from the reaction solution,
c) the combined mother and wash liquors are dehydrated to a water content of $\leq 1.0$ % by distillation,
d) the alkali metal halide which has precipitated is filtered off from the mother liquor and
e) the mother liquor is reused as the reaction medium.

**2.** The process as claimed in claim 1, wherein the sodium or potassium salt of the weak acid is added before the reaction or during recycling of the mother liquor before the filtration (d) or the dehydration (c).

**3.** The process as claimed in claim 1 or 2, wherein the sodium or potassium salt of the weak acid is added as a salt of an aliphatic or aromatic carboxylic acid, in particular acetic acid, propionic acid, butyric acid or benzoic acid.

**4.** The process as claimed in claim 1 or 2, wherein the sodium or potassium salt of the weak acid in the reaction medium is sodium acetate or potassium acetate, which is formed by addition of sodium carbonate or hydroxide or potassium carbonate or hydroxide to the solvent acetic acid.

**5.** The process as claimed in claim 1 or 2, wherein sodium acetate is preferably added for oxidation of a chlorinated alkylaromatic.

**6.** The process as claimed in claim 1 or 2, wherein potassium acetate is preferably added for oxidation of a brominated alkylaromatic.

**7.** The process as claimed in at least one of claims 1 to 6, wherein a compound which contains one or more of the metals cobalt, manganese, zirconium or nickel, in particular cobalt, is employed as the transition metal bromide catalyst.

**8.** The process as claimed in at least one of claims 1 to 7, wherein the reaction solution is cooled to 10 to 60°C, in particular 20 to 30°C, when the reaction has ended.

**9.** The process as claimed in at least one of claims 1 to 8, wherein a substituted or unsubstituted phenyl or naphthyl is employed as the aromatic compound.

**10.** The process as claimed in at least one of claims 1 to 9, wherein a compound of the formula (I)

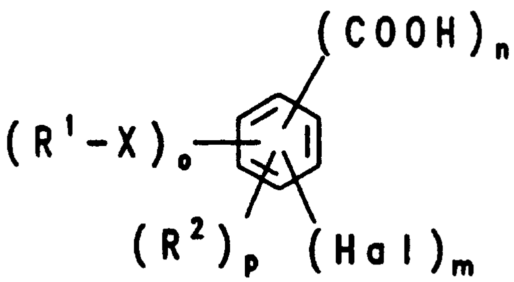

(I)

in which:

X is a single bond, oxygen, C = O, $SO_2$, $C(CF_3)_2$, $C(CH_3)_2$, PO-($C_1$-$C_6$)-alkyl, PO-phenyl, Si-($C_1$-$C_6$-alkyl)$_2$, Si-($C_1$-$C_6$-alkyl)-phenyl, Si-(phenyl)$_2$ or -$N_2$-,
$R^1$ is substituted or unsubstituted phenyl or naphthyl,
Hal is chlorine or bromine,
$R^2$ is hydrogen, O-($C_1$-$C_6$)-alkyl, tert-alkyl, $SO_2$-($C_1$-$C_6$)-alkyl, $SO_2$-$NH_2$, $NO_2$, F, PO-[($C_1$-$C_6$)-alkyl]$_2$, PO(OH)-($C_1$-$C_6$)-alkyl or PO-(OH)$_2$,
n and m are an integer between 1 and 4, where $n + m \leq 5$, and
o and p are zero or 1, where $n+m+o+p \leq 6$, is prepared as the aromatic carboxylic acid.

**11.** A process as claimed in at least one of claims 1 to 8, wherein a chlorobenzoic acid, dichlorobenzoic acid, fluorobenzoic acid, bromobenzoic acid, chlorinated or brominated benzophenonecarboxylic acid or chlorinated or brominated biphenylcarboxylic acid is prepared.

**Revendications**

**1.** Procédé de préparation d'acides carboxyliques aromatiques substitués par des atomes de brome ou de chlore par oxydation des composés alkylaromatiques halogénés correspondants avec de l'oxygène ou un gaz contenant de l'oxygène dans le solvant acide acétique et en présence d'un catalyseur de bromure de métal de transition, caractérisé en ce que

a) le mélange réactionnel contient un sel de sodium et/ou de potassium d'un acide faible,
b) on refroidit la solution de réaction après la fin de la réaction et on filtre le produit cristallisé dans la solution de réaction,
c) on déshydrate les bains de solution mère et de lavage réunis par distillation à une teneur en eau ≤ 1,0%,
d) on filtre l'halogénure alcalin précipité dans la solution mère et
e) on réutilise la solution mère comme milieu réactionnel.

**2.** Procédé selon la revendication 1, caractérisé en ce qu'on ajoute le sel de sodium ou de potassium de l'acide faible avant la réaction, ou lors du recyclage de la solution mère avant la filtration (d) ou la déshydratation (c).

**3.** Procédé selon la revendication 1 ou 2, caractérisé en ce qu'on ajoute les sels de sodium ou de potassium de l'acide faible sous la forme de sels d'acides carboxylique aliphatiques ou aromatiques, en particulier de l'acide acétique, de l'acide propionique, de l'acide butyrique ou de l'acide benzoïque.

**4.** Procédé selon la revendication 1 ou 2, caractérisé en ce que les sels de sodium et de potassium de l'acide faible dans le milieu réactionnel sont l'acétate de sodium ou de potassium, qui sont formés par addition des carbonates ou des hydroxydes de sodium ou de potassium au solvant acide acétique.

**5.** Procédé selon la revendication 1 ou 2, caractérisé en ce qu'on ajoute pour l'oxydation des composés alkylaromatiques chlorés de préférence de l'acétate de sodium.

**6.** Procédé selon la revendication 1 ou 2, caractérisé en ce qu'on ajoute pour l'oxydation des composés alkylaromatiques bromés de préférence de l'acétate de potassium.

**7.** Procédé selon au moins une des revendications 1 à 6, caractérisé en ce qu'on utilise comme catalyseur de bromure de métal de transition un composé, qui contient un ou plusieurs des métaux cobalt, manganèse, zirconium ou nickel, en particulier le cobalt.

**8.** Procédé selon au moins une des revendications 1 à 7, caractérisé en ce qu'on refroidit la solution de réaction après la fin de la réaction à 10 à 60°C, en particulier à 20 à 30°C.

**9.** Procédé selon au moins une des revendications 1 à 8, caractérisé en ce qu'on utilise comme composés aromatiques des groupes phényle ou napthyle substitués ou non substitués.

**10.** Procédé selon au moins une des revendications 1 à 9, caractérisé en ce qu'on prépare comme acides carboxyliques aromatiques des composés de formule (I)

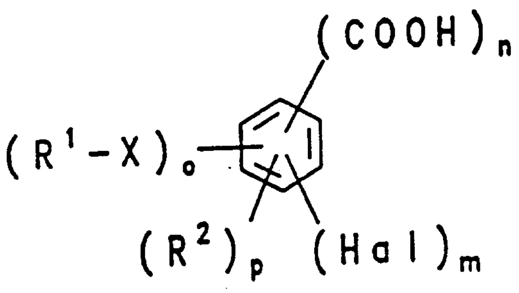

(I)

dans laquelle

x représente une simple liaison, un atome d'oxygène, un groupe C=O, $SO_2$, $C(CF_3)_2$, $C(CH_3)_2$, PO-alkyle en $c_1$-$c_6$, PO-phényle, Si-(alkyle en $C_1$-$C_6$)$_2$, Si-(alkyle en $C_1$-$C_6$)-phényle, Si-(phényle)$_2$, -$N_2$-

$R^1$ représente un groupe phényle, naphtyle substitué ou non substitué,

Hal représente un atome de chlore ou de brome,

$R^2$ représente un atome d'hydrogène, un groupe O-alkyle en $C_1$-$C_6$, tert.-alkyle, $SO_2$-alkyle en $C_1$-$C_6$, $SO_2$-$NH_2$, $NO_2$, F, PO-[alkyle en $C_1$-$C_6$)$_2$, PO(OH)-alkyle en $C_1$-$C_6$, PO-(OH)$_2$,

n, m un nombre entier entre 1 et 4, $n + m \leq 5$ ,

o, p zéro ou 1, tels que $n + m + o + p \leq 6$ .

**11.** Procédé selon au moins une des revendications 1 à 8, caractérisé en ce qu'on prépare des acides chlorobenzoïques, des acides dichlorobenzoïques, des acides fluorobenzoïques, des acides bromobenzoïques, des acides benzophénonecarboxyliques chlorés et bromés, des acides biphénylecarboxyliques chlorés et bromés.